(19) Europäisches Patentamt
European Patent Office
Office européen des brevets

(11) EP 2 436 373 A1

(12) **EUROPEAN PATENT APPLICATION**
published in accordance with Art. 153(4) EPC

(43) Date of publication:
**04.04.2012 Bulletin 2012/14**

(21) Application number: **10780252.2**

(22) Date of filing: **25.05.2010**

(51) Int Cl.:
***A61K 8/81*** *(2006.01)* ***A61K 8/34*** *(2006.01)*
***A61K 8/898*** *(2006.01)* ***A61Q 5/06*** *(2006.01)*
***C08F 20/56*** *(2006.01)* ***C08F 220/10*** *(2006.01)*
***C08F 220/56*** *(2006.01)*

(86) International application number:
**PCT/JP2010/003481**

(87) International publication number:
**WO 2010/137291 (02.12.2010 Gazette 2010/48)**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **28.05.2009 JP 2009128595**

(71) Applicant: **Kao Corporation**
**Chuo-Ku**
**Tokyo 103-8210 (JP)**

(72) Inventors:
- **TATE, Yoshimasa**
  **Tokyo 131-8501 (JP)**
- **YUI, Koji**
  **Tokyo 131-8501 (JP)**
- **ISHII, Keiko**
  **Tokyo 131-8501 (JP)**

(74) Representative: **HOFFMANN EITLE**
**Patent- und Rechtsanwälte**
**Arabellastraße 4**
**81925 München (DE)**

(54) **HAIRSPRAY**

(57) A hairspray which contains (A) to (C):
(A) a cationic coating film-forming polymer, which is a copolymer containing monomers represented by formulae (1) to (4) (30 to 80% of (1), 2 to 50% of (2), 0 to 30% of (3), and 0 to 40% of (4)), or a copolymer including monomers represented by formulae (5) to (8) (30 to 80% of (5), 5 to 45% of (6), 2 to 30% of (7), and 0 to 30% of (8)):

(1)
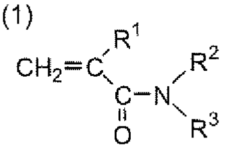

(2)
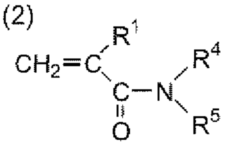

(5)
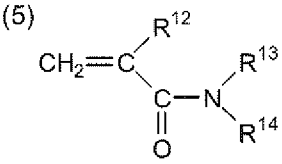

(6)
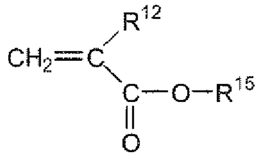

EP 2 436 373 A1

(3)

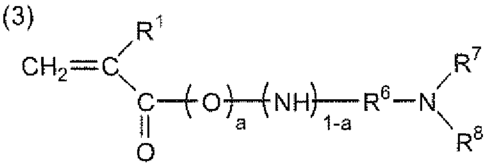

(7)

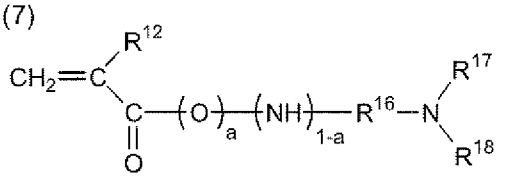

(4)

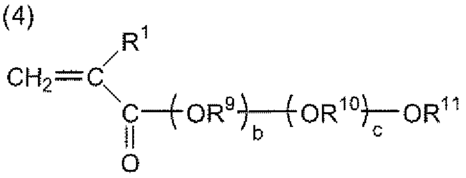

(8)

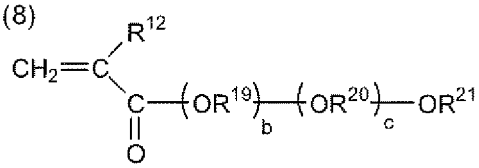

wherein $R^1$, $R^4$, $R^5$, $R^{12}$, and $R^{21}$ are H or lower alkyl; $R^2$, $R^3$, $R^{13}$, and $R^{14}$ are H or $C_{4-12}$ alkyl, or the like; $R^6$, $R^{16}$, $R^9$, $R^{10}$, $R^{19}$, and $R^{20}$ are lower alkylene; $R^7$, $R^8$, $R^{17}$, $R^{18}$, and $R^{15}$ are lower alkyl; $R^{11}$ is H, $C_{1-10}$ alkyl or Ph; "a" is 0 or 1; "b" and " c" are 0 to 50;
(B) an anionic coating film-forming polymer; and
(C) a liquid solvent which has two or more OH and has a molecular weight of 62 to 1000,
and wherein a mass ratio (B)/(A) is from 0.1 to 100.

## Description

Field of the Invention

The present invention relates to a hairspray.

Background of the Invention

In order to fix a hairstyle for about several hours, hairsprays are widely used. The hairsprays are roughly classified into two tyres: one is a type that once fixed hairstyle cannot be fixed again after the hairstyle is out of shape (hereinafter, referred to as a "fixative type") ; and the other is a type that once fixed hair style can be fixed again after the hairstyle is out of shape (hereinafter, referred to as "viscous type") (for example, Patent Document 1).

The fixative type hairspray forms a polymer coating film which provides a strong ability for hair setting, however, on the other hand, this type of hairspray involves a problem such that the coating film generates feel of stiffness, and that a hairstyle cannot be fixed again when the coating film is damaged (hereinafter, the "fixing again" is referred to as "hair-re-styling"). On the contrary, since the viscous type hairspray fixes a hairstyle by viscosity derived from a polymer for hairdressing and a plasticizer, even if the hairstyle is out of shape, the hairstyle can be fixed again and again. However, when high viscosity is provided in order to firmly fix the hairstyle, a problem of stickiness may occur accordingly. Therefore, in the case of using viscous type hairsprays having high viscosity, special using methods are purposely proposed (see, for example, Patent Document 2). Meanwhile, in the case of the degree of viscosity not enough to feel stickiness, ability for fixing the hairstyle is weak. Thus, no hairspray which is capable of strongly fixing a hairstyle, capable of hair-re-styling, and provides less stickiness, has been existed.

Patent Document

[Patent Document 1] JP-A-2005-68134
[Patent Document 2] JP-A-2009-7347

Summary of the Invention

The present invention provides a hairspray which contains the following components (A) to (C), wherein a mass ratio (B)/(A) of the component (B) to the component (A) is from 0.1 to 100.

(A) One or more cationic coating film-forming polymers selected from (A1) and (A2):

(A1) a copolymer obtained from a monomer mixture including following monomers (a) to (d),

(a) 30 to 80 mass% of a (meth)acrylamide monomer represented by a formula (1):

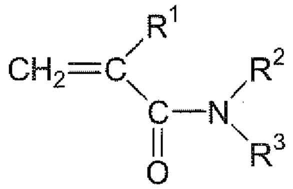

(1)

wherein $R^1$ is a hydrogen atom or a methyl group, $R^2$ and $R^3$ are the same as or different from each other and are a hydrogen atom or an alkyl group having 4 to 12 carbon atoms, with the proviso that both $R^2$ and $R^3$ are not a hydrogen atom simultaneously,

(b) 2 to 50 mass% of a (meth)acrylamide monomer represented by a formula (2):

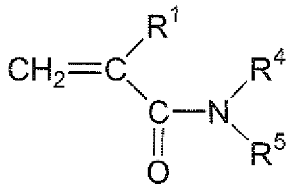

(2)

wherein $R^1$ is the same as mentioned above, $R^4$ and $R^5$ are the same as or different from each other and are a hydrogen atom or an alkyl group having 1 to 3 carbon atoms,
(c) 0 to 30 mass% of a (meth)acrylic ester monomer or a (meth)acrylamide monomer represented by a formula (3):
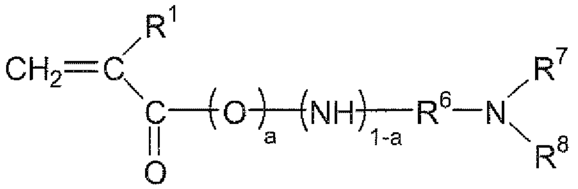

(3)

wherein $R^1$ is the same as mentioned above, $R^6$ is an alkylene group having 2 or 3 carbon atoms, $R^7$ and $R^8$ are the same as or different from each other and are a methyl group or an ethyl group, and "a" is a number of 0 or 1, and
(d) 0 to 40 mass% of a (meth)acrylic ester monomer represented by a formula (4):
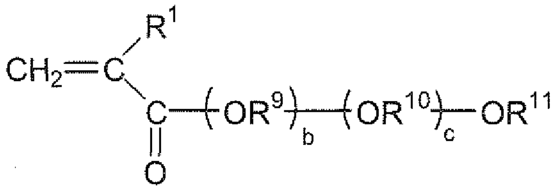

(4)

wherein $R^1$ is the same as mentioned above, $R^9$ and $R^{10}$ are the same as or different from each other and are an alkylene group having 2 to 4 carbon atoms, $R^{11}$ is a hydrogen atom, an alkyl group having 1 to 10 carbon atoms, or a phenyl group, and "b" and "c" are independently a number of from 0 to 50, with the proviso that both "b" and "c" are not 0 simultaneously; and (A2) a copolymer obtained from a monomer mixture including following monomers (e) to (h),
(e) 30 to 80 mass% of a (meth)acrylamide monomer represented by a formula (5):
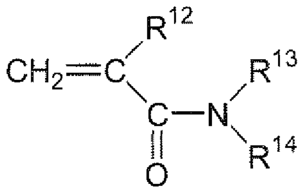

(5)

wherein $R^{12}$ is a hydrogen atom or a methyl group, $R^{13}$ and $R^{14}$ are the same as or different from each other and are a hydrogen atom or an alkyl group having 4 to 12 carbon atoms, or $R^{13}$ and $R^{14}$ are combined together with an adjacent nitrogen atom to form a ring,
(f) 5 to 45 mass% of a (meth)acrylic ester monomer represented by a formula (6):
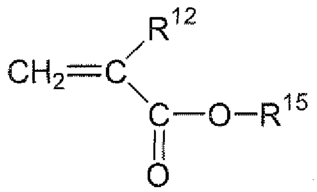

(6)

wherein $R^{12}$ is the same as mentioned above, and $R^{15}$ is an alkyl group having 1 to 4 carbon atoms,
(g) 2 to 30 mass% of a (meth)acrylic ester monomer or a (meth)acrylamide monomer represented by a formula (7) :
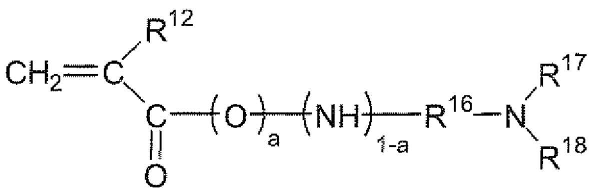

(7)

wherein $R^{12}$ is the same as mentioned above, $R^{16}$ is an alkylene group having 2 or 3 carbon atoms, $R^{17}$ and $R^{18}$ are the same as or different from each other and are a methyl group or an ethyl group, and "a" is a number of 0 or 1, and
(h) 0 to 30 mass% of a (meth)acrylic ester monomer represented by a formula (8):
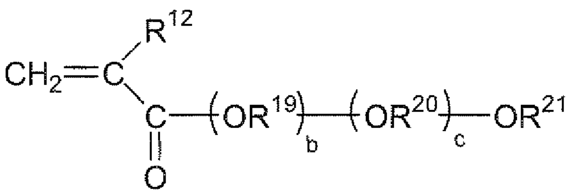

(8)

wherein $R^{12}$ is the same as mentioned above, $R^{19}$ and $R^{20}$ are the same as or different from each other and are an alkylene group having 2 to 4 carbon atoms, $R^{21}$ is a hydrogen atom or a methyl group, and "b" and "c" are independently a number of from 0 to 50, with the proviso that both "b" and "c" are not 0 simultaneously;

(B) an anionic coating film-forming polymer; and
(C) a solvent which has two or more hydroxyl groups, has a molecular weight of 62 or more and 1000 or less, and is in a liquid state at 30°C.

Detailed Description of the Invention

The present invention relates to a hairspray which is capable of strongly fixing a hairstyle, provides less sticky feeling, and capable of re-styling of hair style.
The inventors of the present invention have found that the above-mentioned problems are solved by using a certain cationic coating film-forming polymer and a certain anionic coating film-forming polymer in a specific ratio, and further using a specific solvent.
In the present invention, the "coating film-forming polymer" is a polymer having a breaking strength of 100 gf/cm$^2$ or more, preferably 200 gf/cm$^2$ or more, and even more preferably 300 gf/cm$^2$ or more, as measured by the below-mentioned measuring method.

Measuring Method

<Preparation of Sample>

(1) Place 15 g of an ethanol solution of a polymer to be evaluated at such a concentration that a coating film mentioned below having a thickness of 200 to 400 μm is formed in a 7.5 cm diameter Petri dish (made of Teflon (R)), and evaporate at 25°C, 65%Rh for three days to dryness.
(2) Place the same Petri dish in a thermostat box set at 25°C, 98%Rh for 30 min.
(3) Separate the softened coating film from the Petri dish with, for example, a spatula.
(4) Repeat the operations (2) and (3) if the coating film cannot be separated.
(5) Cut the coating film into pieces of 3.0 mm x 30.0 mm.
(6) Array the cut pieces of the coating films on a Teflon (R) sheet without overlapping each other, further lay a Teflon (R) sheet and a plastic sheet. Then, place a weight on the plastic sheet to prevent the film from warping during drying.
(7) A day later, measure the thickness of the coating film at the center portion thereof with a thickness gauge ("SMD-565", manufactured by TECLOCK Corporation) (N = 5).

<Measurement of Breaking Strength of Sample>

(1) Place the cut out pieces of the coating film on a Teflon (R) sheet, and place the pieces of the coating film to stand still for two hours in a thermostat box set at 20°C, 98%Rh.
(2) Take out the pieces of the coating film from the thermostat box and measure the breaking strength of the film immediately by using a tensile and compression testing machine ("TMD-200N" manufactured by Minebea Co., Ltd.) (chuck to chuck distance: 2 cm, sample length between chucks: 0.5 cm, pulling rate: 200 mm/min).
(3) Calculate the cross-sectional area of the coating film based on the thickness of the coating film measured previously.
(4) Calculate a breaking strength by dividing the coating film breaking strength by the cross-sectional area (N = 5).

[(A): Cationic Coating Film-Forming Polymers]

Among the cationic coating film-forming polymers of the component (A) used in the present invention, examples of (a) (meth)acrylamide monomer, represented by the formula (1) in (A1), include N-n-butyl (meth)acrylamide, N-tert-butyl (meth)acrylamide, N-octyl (meth)acrylamide, N-lauryl (meth)acrylamide, N-1-methylundecyl (meth)acrylamide, N-2-ethylhexyl (meth)acrylamide, and N-tert-octyl (meth)acrylamide. Among them, N-branched alkyl (meth)acrylamides such as N-tert-butyl (meth)acrylamide, N-tert-octyl (meth)acrylamide, and N-2-ethylhexyl (meth)acrylamide are preferred. These may be used alone or in combination of two or more, in an amount of from 30 to 80 mass%, preferably from 40 to 70 mass% of whole monomers.
Examples of (b) (meth)acrylamide monomer, represented by the formula (2), include (meth)acrylamide, N-methyl (meth)acrylamide, N-ethyl (meth)acrylamide, N-isopropyl (meth)acrylamide, N,N-dimethyl (meth)acrylamide, and N,N-diethyl (meth)acrylamide. Among them, N-methyl (meth)acrylamide, N-ethyl (meth)acrylamide, N,N-dimethyl (meth) acrylamide, and N,N-diethyl (meth)acrylamide are preferred.
These may be used alone or in combination of two or more, in an amount of from 2 to 50 mass%, preferably from 10 to 35 mass% of whole monomers.
Examples of (c) (meth)acrylic ester and (meth)acrylamide monomer, represented by the formula (3), include N,N-dimethylaminoethyl (meth)acrylate, N,N-diethylaminoethyl (meth)acrylate, N,N-dimethylaminopropyl (meth)acrylate, N,N-dimethylaminopropyl (meth)acrylamide, and N,N-diethylaminopropyl (meth)acrylamide.
These may be used alone or in combination of two or more in an amount of from 0 to 30 mass%, preferably from 0 to 10 mass%, more preferably from 0.5 to 5 mass%, of whole monomers.
The monomer (d) represented by the formula (4) is a (meth)acrylic ester having a polyoxyalkylene chain. In the formula, $R^{11}$ is preferably a hydrogen atom or an alkyl group having 1 to 4 carbon atoms, and more preferably, a methyl group. Examples of such a (meth)acrylic ester monomer (4) include hydroxyethyl (meth)acrylate, hydroxypropyl (meth)acrylate, polyethylene glycol mono(meth)acrylate, polypropylene glycol mono(meth)acrylate, methoxypolyethylene glycol mono(meth)acrylate, methoxypolypropylene glycol mono(meth)acrylate, ethoxypolyethylene glycol mono (meth)acrylate, butoxypolyethylene glycol mono(meth)acrylate, and phenoxypolyethylene glycol mono(meth)acrylate. The polyoxyalkylene chain is a homopolymer or copolymer of $C_{2-4}$ alkylene oxide. In the case of a copolymer, it may be

a block copolymer or a random copolymer of, for example, ethylene oxide, propylene oxide. The degree of polymerization of the alkylene oxide can be analyzed by using gas chromatography and it is preferably from 1 to 50 on average.
These monomers may be used alone or in combination of two or more in an amount of from 0 to 40 mass%, preferably from 5 to 30 mass%, more preferably from 10 to 25 mass%, of whole monomers.
Preferred examples of the copolymer (A1) include:

N-tert-butyl (meth)acrylamide/N,N-dimethyl (meth)acrylamide/N,N-dimethylaminopropyl (meth)acrylamide/methoxypolyethylene glycol (meth)acrylate,
N-tert-butyl (meth)acrylamide/N,N-dimethyl (meth)acrylamide/N,N-dimethylaminoethyl (meth)acrylate/methoxypolyethylene glycol (meth)acrylate,
N-tert-butyl (meth)acrylamide/N-methyl (meth)acrylamide/N,N-dimethylaminoethyl (meth)acrylate/methoxypolyethylene glycol (meth)acrylate,
N-tert-butyl (meth)acrylamide/N-methyl (meth)acrylamide/N,N-dimethylaminopropyl (meth)acrylamide/methoxypolyethylene glycol (meth)acrylate,
N-tert-butyl (meth)acrylamide/N,N-dimethyl (meth)acrylamide/N,N-dimethylaminopropyl (meth)acrylamide,
N-tert-octyl (meth)acrylamide/N,N-diethyl (meth)acrylamide/N,N-dimethylaminopropyl (meth)acrylamide/methoxypolyethylene glycol (meth)acrylate,
N-tert-octyl (meth)acrylamide/N,N-dimethyl (meth)acrylamide/N,N-dimethylaminopropyl (meth)acrylamide/methoxypolyethylene glycol (meth)acrylate,
N-tert-butyl (meth)acrylamide/N,N-dimethyl (meth)acrylamide/N,N-dimethylaminopropyl (meth)acrylamide/2-hydroxyethyl (meth)acrylate, and
N-tert-butyl (meth)acrylamide/N,N-diethyl (meth)acrylamide/N,N-dimethylaminopropyl (meth)acrylamide/methoxypolyethylene glycol (meth)acrylate.
N-tert-butyl (meth)acrylamide/N,N-dimethyl (meth)acrylamide/N,N-dimethylaminopropyl (meth)acrylamide/methoxypolyethylene glycol (meth)acrylate is more preferred.

Furthermore, examples of (e) the (meth)acrylamide monomer represented by the formula (5) in (A2) include (meth)acrylamide, N-n-butyl (meth)acrylamide, N-tert-butyl (meth)acrylamide, N-octyl (meth)acrylamide, N-lauryl (meth) acrylamide, and (meth)acryloylmorpholine. Among them, N-butyl (meth)acrylamide, N-octyl (meth)acrylamide, and N-lauryl (meth)acrylamide are preferred.
These may be used alone or in combination of two or more in an amount of from 30 to 80 mass%, preferably from 40 to 70 mass%, of whole monomers.
Examples of (f) the (meth)acrylic ester monomer represented by the formula (6) include methyl (meth)acrylate, ethyl (meth)acrylate, propyl (meth)acrylate, and butyl (meth)acrylate.
These may be used alone or in combination of two or more in an amount of from 5 to 45 mass%, preferably from 10 to 40 mass% of whole monomers.
Examples of (g) the (meth)acrylic ester and (meth)acrylamide monomer represented by the formula (7) include N,N-dimethylaminoethyl (meth)acrylate, N,N-diethylaminoethyl (meth)acrylate, N,N-dimethylaminopropyl (meth)acrylate, N,N-dimethylaminopropyl (meth)acrylamide, and N,N-diethylaminopropyl (meth)acrylamide.
These may be used alone or in combination of two or more in an amount of from 2 to 30 mass%, preferably from 5 to 20 mass% of whole monomers.
Examples of (h) the (meth)acrylic ester monomer represented by the formula (8) include hydroxyethyl (meth) acrylate, hydroxypropyl (meth)acrylate, polyethylene glycol mono(meth)acrylate, polypropylene glycol mono(meth)acrylate, methoxypolyethylene glycol mono(meth)acrylate, and methoxy polypropylene glycol mono(meth)acrylate. The polyoxyalkylene chain is a homopolymer or a copolymer of $C_{2-4}$ alkylene oxide. In the case of a copolymer, it may be a block copolymer or a random copolymer of, for example, ethylene oxide, propylene oxide. The degree of polymerization of the alkylene oxide can be analyzed by using gas chromatography and it is preferably from 1 to 50 on average.
These may be used alone or in combination of two or more in an amount of from 0 to 30 mass%, preferably from 5 to 15 mass%, of whole monomers.
Preferred examples of the copolymer (A2) include
N-tert-butyl (meth)acrylamide/N,N-dimethylaminopropyl (meth)acrylamide/methoxypolyethylene glycol (meth)acrylate/ethyl (meth)acrylate,
N-tert-butyl (meth)acrylamide/N,N-dimethylaminoethyl (meth)acrylate/methoxypolyethylene glycol (meth)acrylate/ethyl (meth)acrylate,
N-tert-butyl (meth)acrylamide/N,N-dimethylaminoethyl (meth)acrylate/methoxypolyethylene glycol (meth)acrylate/methyl (meth)acrylate,
N-tert-butyl (meth)acrylamide/N,N-diethylaminoethyl
(meth)acrylate/methoxypolyethylene glycol

(meth)acrylate/ethyl(meth)acrylate,
N-tert-butyl (meth)acrylamide/N,N-dimethylaminoethyl (meth)acrylate/2-hydroxyethyl (meth)acrylate/ethyl (meth)acrylate,
N-tert-octyl (meth)acrylamide/N,N-dimethylaminoethyl (meth)acrylate/methoxypolyethylene glycol (meth)acrylate/n-butyl (meth)acrylate,
N-tert-octyl (meth)acrylamide/N,N-diethylaminoethyl (meth)acrylate/methoxypolyethylene glycol (meth)acrylate/n-butyl (meth)acrylate, and
N-tert-butyl (meth)acrylamide/N,N-dimethylaminopropyl (meth)acrylamide/ethyl (meth)acrylate.
Among them, N-tert-butyl (meth)acrylamide/N,N-dimethylaminopropyl (meth)acrylamide/methoxypolyethylene glycol (meth)acrylate/ethyl (meth)acrylate is more preferred.

The copolymers (A1) and (A2) can each be prepared by using the monomers described above in combination in accordance with the process described in, for example, JP-A-08-291206 or JP-A-02-180911.

A weight average molecular weight (measured by gel filtration chromatography (relative to polyethylene glycol)) of the copolymer thus obtained can be controlled in the range of from 1,000 to 1,000,000 by selection of the polymerization conditions. In the present invention, preferable weight average molecular weight of the copolymer is from 10,000 to 500,000, more preferably from 20,000 to 200,000.

The copolymers thus obtained may be used after neutralization of their tertiary amino group with an inorganic acid or organic acid, in order to impart water solubility to the copolymers. In this case, preferably 50% or more of total tertiary amino groups are neutralized.

Examples of the inorganic acid include hydrochloric acid, sulfuric acid, and phosphoric acid. Examples of the organic acid include acetic acid, glycolic acid, dimethylglycolic acid, lactic acid, dimethylolpropionic acid, tartaric acid, citric acid, maleic acid, and malic acid.

The amino group in the copolymer may be quaternized with an appropriate quaternizing agent. In this case, preferably 50% or more of total tertiary amino groups are quaternized.

Examples of such a quaternizing agent include dialkyl sulfates such as dimethyl sulfate and diethyl sulfate, alkyl halides such as methyl chloride, propyl bromide, and benzyl chloride, and aralkyl halides.

Such a quaternized copolymer can also be obtained by quaternizing the monomer (3) or (7) with a quaternizing agent, followed by copolymerization.

One or more polymer(s) selected from (A1) and (A2) can be used as the cationic coating film-forming polymer of the component (A). The content of the film-forming polymer(s) in the whole composition of the hairspray is preferably from 0.3 to 10 mass%, more preferably from 0.5 to 8 mass%, and even more preferably 0.7 to 7 mass% from the viewpoint of obtaining high hair-setting ability and hair-re-styling property. In the case that the hairspray of the present invention is an aerosol hairspray containing an aerosol stock solution and a propellant, insofar as the content of each component is discussed, "whole composition" means a whole composition of the aerosol stock solution without containing a propellant.

[(B): Anionic Coating Film-Forming Polymer]

Examples of the anionic coating film-forming polymer of the component (B) include the following.

- Anionic coating film-forming polymers containing, as a structural unit thereof, crotonic acid, maleic acid, maleic acid monoester or itaconic acid:

  The embodiment is not particularly limited as far as it contains, as a structural unit thereof, crotonic acid, maleic acid, maleic acid monoester or itaconic acid, those obtained by copolymerization with one or more of vinyl ethers, vinyl esters, and vinyl alcohols are preferred; methyl vinyl ether/alkyl maleate copolymers (for example, "Gantrez ES-225", "Gantrez ES-425", and "Gantrez SP-215", each manufactured by ISP), vinyl acetate/crotonic acid copolymers (for example, "Resyn 28-1310" manufactured by National Starch), vinyl acetate/crotonic acid/vinyl neodecanoate copolymers (for example, "Resyn 28-2930" manufactured by National Starch), vinyl acetate/crotonic acid/vinyl propionate copolymers (for example, "Luviset CAP" manufactured by BASF), and vinyl alcohol/itaconic acid copolymers (for example, "KM-118" manufactured by Kuraray) are more preferred; and methyl vinyl ether/alkyl maleate copolymers, vinyl acetate/crotonic acid copolymers, vinyl acetate/crotonic acid/vinyl neodecanoate copolymers, and vinyl acetate/crotonic acid/vinyl propionate copolymers are even more preferred.

- Anionic coating film-forming polymers containing, as a structural unit thereof, (meth)acrylic acid:

The embodiment is not particularly limited as far as it is a vinyl polymer containing, as a structural unit thereof, (meth)acrylic acid, copolymers with one or more of alkyl (meth)acrylate esters and N-alkyl (meth)acrylamides are preferred.

More preferred examples include acrylic acid/ethyl acrylate/N-t-butyl acrylamide copolymers (for example, "Ultrahold 8" and "Ultrahold Strong", each manufactured by BASF), octyl acrylamide/acrylic acid copolymers (for example, "Amphomer V-42" manufactured by National Starch), acrylate/methacrylate/acrylic acid/methacrylic acid copolymers (for example, "Amerhold DR25" manufactured by Union Carbide), acrylates/diacetoneacrylamide copolymers (for example, "Pluscize L-9540B" manufactured by Goo Chemical (neutralized product of this polymer with 2-amino-2-methyl-1-propyl alcohol)), and acrylates/$C_{1-18}$ alkyl acrylates/$C_{1-8}$ alkylacrylamide copolymers (for example, "Pluscize L-9909B" manufactured by Goo Chemical (neutralized product of this polymer with 2-amino-2-methyl-1-propyl alcohol)), and acrylate/methacrylate/acrylic acid/methacrylic acid copolymers, acrylates/diacetoneacrylamide copolymers, and acrylates/$C_{1-18}$ alkyl acrylates/$C_{1-8}$ alkylacrylamide copolymers are even more preferred.

- Natural anionic coating film-forming polymers having carboxyl group or sulfuric acid group:

The embodiment is not particularly limited as far as it is a natural polysaccharide having either a carboxyl group or a sulfuric acid group, preferred examples include carrageenan (for example, "Soageena LX22" and "Soageena ML210" manufactured by Mitsubishi Rayon) and xanthan gum (for example, "ECHO gum T", manufactured by Dainippon Sumitomo Pharma Co., Ltd.), and carrageenan is more preferred.

- Polyesters having carboxyl group or sulfonic acid group:

The embodiment is not particularly limited and water dispersible polyesters (for example, "AQ38S" and "AQ55S" manufactured by Eastman Kodak) are preferred.

As the anionic coating film-forming polymer, methyl vinyl ether/alkyl maleate copolymers, acrylates/diacetoneacrylamide copolymers, and acrylates/$C_{1-8}$ alkyl acrylates/$C_{1-8}$ alkylacrylamide copolymers are preferred.

One or more of the above mentioned anionic coating film-forming polymers can be used as the component (B). From the viewpoint of obtaining both high hair setting ability and non-stiffness, the content thereof in the whole composition is preferably from 0.5 to 12.0 mass%, more preferably from 1.0 to 10.0 mass%, even more preferably from 1.5 to 8.0 mass%.

Furthermore, the component (A) and the component (B) are contained at a mass ratio (B)/(A) of preferably from 0.1 to 100, more preferably from 0.25 to 20, even more preferably from 0.4 to 10, and even more preferably from 1 to 5, from the viewpoint of keeping balance between high hair setting ability, high wash-off property, non-stiffness, non-stickiness, less whitening, and compatibility with a propellant. The term "whitening" as used herein means a phenomenon that when the hairspray is sprayed to hair, the hair seems white due to the irregular reflection of light, which is caused by air bubbles contained in the composition applied to the hair.

[(C): Solvent Having Two or More Hydroxyl Groups]

The solvent of the component (C) is a solvent which has two or more hydroxyl groups, a molecular weight of 62 or more and 1000 or less, and is in a liquid state at 30°C or less, and preferably the molecular weight is 90 or more and 600 or less, and more preferably 100 or more and 200 or less. Specific examples thereof may include, for example, propylene glycol (molecular weight: 76.1), 1,3-butylene glycol (molecular weight: 90.1), glycerin (molecular weight: 92.1), isopentylene glycol (molecular weight: 104.1), hexylene glycol (molecular weight: 118.2), dipropylene glycol (molecular weight: 134.2), polypropylene glycol (degree of polymerization: 9, molecular weight: 540), and polyethylene glycol 600. Among them, from the viewpoint of obtaining an appropriate tackiness, molecules having 4 or more carbon atoms are preferred. Specifically, dipropylene glycol, polypropylene glycol (degree of polymerization: 9, molecular weight: 540), and 1,3-butylene glycol are preferred, dipropylene glycol is more preferred. The term "in a liquid state" as used herein means a state in which the viscosity is 1000 mPa·s or less when measured by using a Brookfield viscometer (Rotor No. 2, rotation speed at 12 rpm, for 60 seconds at 30°C) (the same as in the component (E)).

These solvents may contain two or more components (C) in combination, and the content thereof is preferably from 1 to 25 mass%, more preferably from 1.5 to 10 mass%, and even more preferably from 2 to 8 mass% with respect

to the whole composition, from the viewpoint that combination of the components (A) and (B) form a sticky coating film to adhere hairs to each other, thus enabling the hairs to be re-styled.

[(D): Poly(N-acylalkyleneimine)-Modified Silicone]

The hairspray of the present invention may further contain poly(N-acylalkyleneimine)-modified silicone as the component (D) in order to reduce stiffness and stickiness of hair to which the hairspray has been applied and to provide appropriate slip properties. Examples of the poly(N-acylalkyleneimine)-modified silicone include organopolysiloxane, wherein a poly(N-acylalkyleneimine) segment containing repeating units, represented by the following formula (9), is linked to, via a heteroatom-containing alkylene group, at least two of silicon atoms in an organopolysiloxane segment which constitutes a main chain:

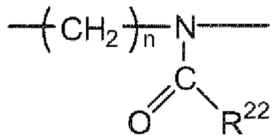

(9)

and wherein, $R^{22}$ is a hydrogen atom, an alkyl group having 1 to 22 carbon atoms, an aralkyl group, or an aryl group, and n denotes 2 or 3,

a number average molecular weight of the poly(N-acylalkyleneimine) segment is 500 to 10,000,

a mass ratio (a/b) of the organopolysiloxane segment (a) constituting the main chain and the poly(N-acylalkyleneimine) segment (b) is 40/60 to 98/2, and

a weight average molecular weight of the organopolysiloxane segment constituting the main chain is 10,000 to 150,000.

In the component (D), at least two of the poly(N-acylalkyleneimine) segments can be bound to arbitrary silicon atoms constituting the organopolysiloxane segment via a heteroatom-containing alkylene group. It is preferable that they are bound to one or more silicon atoms excluding both ends via the above-mentioned alkylene group, and it is more preferable that they are bound to two or more silicon atoms excluding both ends via the above-mentioned alkylene group.

Examples of the heteroatom-containing alkylene group via which the organopolysiloxane segment is bound to poly(N-acylalkyleneimine) include an alkylene group having from 2 to 20 carbon atoms and containing from 1 to 3 nitrogen atoms, oxygen atoms and/or sulfur atoms. Specific examples are as follows:

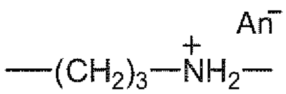
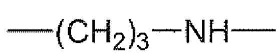
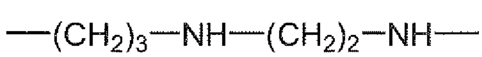

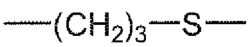
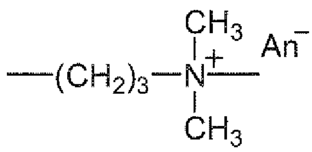
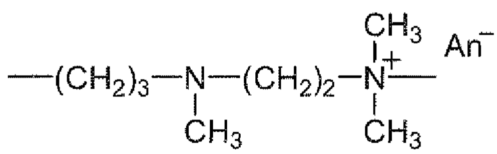

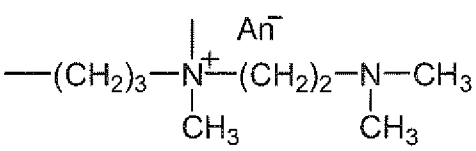
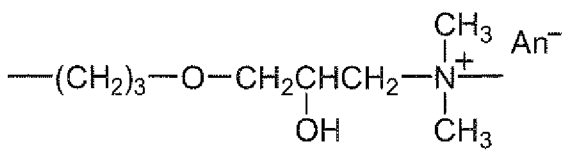

[wherein $An^-$ means anion]

The N-acyl alkylene imine unit constituting the poly(N-acylalkyleneimine) segment is represented by the above-mentioned formula (9). Examples of the alkyl group having 1 to 22 carbon atoms of $R^{22}$ in the formula (9) include a straight-chain, branched-chain, or cyclic alkyl group having 1 to 22 carbon atoms. Specific examples include a methyl group, an ethyl group, an n-propyl group, an isopropyl group, an n-butyl group, a tert-butyl group, a pentyl group, a hexyl group, a cyclohexyl group, a heptyl group, an octyl group, a nonyl group, a decyl group, an undecyl group, a dodecyl group, an octadecyl group, a nonadecyl group, an eicosyl group, and a docosyl group. Among them, an alkyl group having 1 to 10 carbon atoms is preferable, and an alkyl group having 1 to 6 carbon atoms is more preferable.

Examples of the aralkyl group include an aralkyl group having 7 to 15 carbon atoms, and specific examples include a benzyl group, a phenethyl group, a trityl group, a naphthyl methyl group, and an anthracenyl methyl group. Among them, an aralkyl group having 7 to 14 carbon atoms is preferable, and an aralkyl group having 7 to 10 carbon atoms is more preferable.

Examples of the aryl group include an aryl group having 6 to 14 carbon atoms, and specific examples include a phenyl group, a tolyl group, a xylyl group, a naphthyl group, a biphenyl group, an anthryl group, and a phenanthryl group. Among them, an aryl group having 6 to 12 carbon atoms is preferable, and an aryl group having 6 to 9 carbon atoms is more preferable.

Among these, as $R^{22}$, an alkyl group having 1 to 6 carbon atoms is preferable.

The mass ratio (a/b) of the organopolysiloxane segment (a) and the poly(N-acylalkyleneimine) segment (b) is 40/60 to 98/2. From the viewpoints of avoiding stickiness and obtaining excellent setting performance and hair-re-styling performance, the mass ratio is preferably 65/35 to 90/10, more preferably 68/3.3 to 80/20, even more preferably 70/30 to 79/21, and even more preferably 73/27 to 79/21.

Note here that in this specification, the mass ratio (a/b) denotes a value obtained from the integral ratio of an alkyl group or a phenyl group in the organopolysiloxane segment and a methylene group in the poly(N-acylalkyleneimine) segment, the value was measured by dissolving 5 mass% of organopolysiloxane of the component (D) in deuteriochloroform and nuclear magnetic resonance ($^1$H-NMR) analysis.

Furthermore, the weight average molecular weight (MWg) of an organopolysiloxane segment between adjacent poly(N-acylalkyleneimine) segments is preferably from 1,000 to 22,000. From the viewpoints of preventing stickiness and obtaining excellent setting performance and hair-re-styling performance, the weight average molecular weight is preferably from 1,500 to 6,000, more preferably from 1,600 to 4,000, and even more preferably from 1,700 to 3,000.

In this specification, the term "organopolysiloxane segment between adjacent poly(N-acylalkyleneimine) segments" means, as shown in the following formula (2), a part between a connection point to the organopolysiloxane segment of the poly(N-acylalkyleneimine) segment (connection point A) and an adjacent connection point (connection point B) of the poly(N-acylalkyleneimine) segment, surrounded by the broken line, and which is composed of a segment composed of one $R^{23}SiO$ unit, one $R^{27}$, and (y+1) of $R^{23}{}_2SiO$ units. Furthermore, the term "poly(N-acylalkyleneimine) segment" means Z bound to the above-mentioned $R^{27}$.

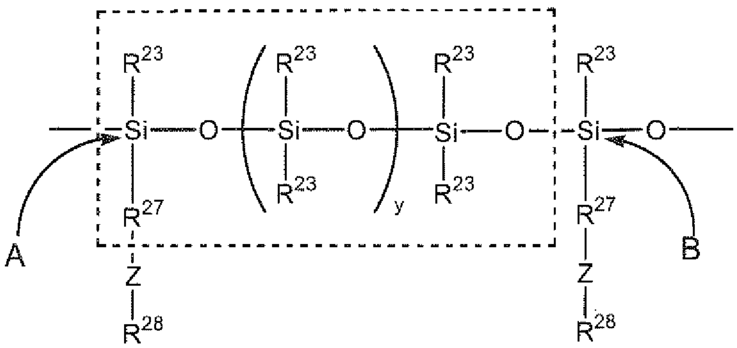

(10)

In the above-mentioned formula (10), $R^{23}$ independently means an alkyl group having 1 to 22 carbon atoms or a phenyl group, $R^{27}$ is a heteroatom-containing alkylene group, Z is a poly(N-acylalkyleneimine) segment, $R^{28}$ is a residue of a polymerization initiator, and y is a positive numeric value.

The MWg means a molecular weight of the part surrounded by a broken line in the above-mentioned formula (10), in another word, means a mass (g/mol) of the organopolysiloxane segment per mole of the poly(N-acylalkyleneimine) segment, which corresponds to a functional group equivalent (g/mol) of a modified organopolysiloxane when 100% of the functional group of the modified organopolysiloxane, which is a raw material compound, is substituted with poly(N-acylalkyleneimine).

The molecular weight of the poly(N-acylalkyleneimine) segment (MWox) can be determined by the method

wherein the value is calculated from molecular weight of the N-acyl alkylene imine unit and the polymerization degree, or the below-mentioned gel permeation chromatography (GPC) measurement method. In the present invention, the molecular weight means a number average molecular weight determined by the GPC measurement method, and the value is from 500 to 10,000, preferably from 800 to 3,000, more preferably 850 to 1,500, and even more preferably from 900 to 1,200, in order to prevent stickiness and to obtain excellent setting performance and hair-re-styling performance.

Furthermore, the MWg can be determined from the below formula by using the content ratio of the organopolysiloxane segment constituting the main chain (Csi).

$$MWg = \frac{Csi \times MWox}{100 - Csi}$$

The weight average molecular weight of the organopolysiloxane segment (MWsi) constituting a main chain is 10,000 to 150,000. From the viewpoint of obtaining sufficient coating strength, solubility in a polar solvent such as water and usability after dissolution, the weight average molecular weight is preferably from 20,000 to 80,000, and more preferably from 30,000 to 40,000. Since the organopolysiloxane segment has a common skeleton to that of the modified organopolysiloxane as a raw material compound, the MWsi is substantially the same as the weight average molecular weight of the modified organopolysiloxane as a raw material compound. In the subject invention, the weight average molecular weight of the modified organopolysiloxane as a raw material compound is measured by GPC in the below-mentioned measurement conditions in terms of polystyrene.

Column: Super HZ4000 + Super HZ2000 (manufactured by TOSOH CORPORATION)
Eluent: 1 mM triethylamine/THF
Flow rate: 0.35 mL/min
Column temperature: 40°C
Detector: UV
Sample: 50 μL

The weight average molecular weight (MWt) of organopolysiloxane of the component (D) is preferably from 10,000 to 500,000, more preferably from 12,000 to 100,000, even more preferably from 24,000 to 80,000, and even more preferably from 35,000 to 45,000, in order to obtain sufficient coating film strength and excellent solubility in a polar solvent such as water, furthermore, in order to prevent stickiness, and to improve both styling performance, that is, setting performance and hair-re-styling performance. In this specification, the MWt can be obtained from the weight average molecular weight of the modified organopolysiloxane as a raw material compound and the above-mentioned mass ratio (a/b).

Organopolysiloxane of the component (D) can be produced by reacting, for example, modified organopolysiloxane represented by the following formula (11) with terminal reactive poly(N-acylalkyleneimine) obtained by ring-opening polymerizing cyclic imino ether represented by the following formula (12).

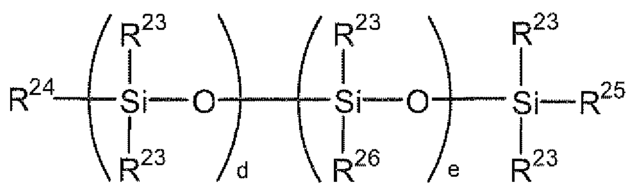

(11)

wherein $R^{23}$ is the same as defined above, $R^{24}$ and $R^{25}$ are the same group as that of $R^{23}$ or a monovalent group represented by any of the following formulae:

$-(CH_2)_3-NH_2$ $-(CH_2)_3-NH-(CH_2)_2-NH_2$

$-(CH_2)_3-SH$

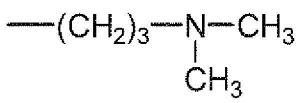

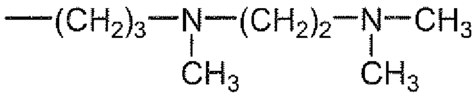

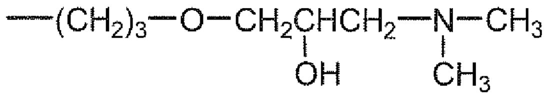

,

wherein $R^{26}$ is a monovalent group represented by the above-mentioned formula, d is an integer from 135 to 1350, and e is an integer from 3 to 57,

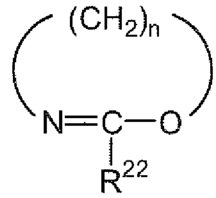

(12)

wherein $R^{22}$ and n are the same as mentioned above.

It is desirable to use modified organopolysiloxane wherein a functional group equivalent is preferably from 1700 to 3500, more preferably from 1800 to 3200, and even more preferably from 2000 to 3000, as well as the weight average molecular weight is preferably from 10,000 to 100,000, more preferably from 20,000 to 80,000, and even more preferably from 30,000 to 60,000.

Furthermore, it is desirable that the molecular weight of the terminal reactive poly(N-acylalkyleneimine) is adjusted to preferably from 500 to 10,000, more preferably from 800 to 3,000, even more preferably from 850 to 1,500, and even more preferably from 900 to 1,200.

A polymerization initiator can be used for the ring-opening polymerization of the cyclic imino ether (12). Examples of the polymerization initiator to be used include a compound having high electrophilic reactivity, for example, alkyl esters of strong acid such as alkyl benzenesulfonate ester, alkyl p-toluenesulfonate ester, alkyl trifluoromethanesulfonate ester, alkyl trifluoroacetate ester, and dialkyl sulfate ester. Among them, dialkyl sulfate can be suitably used. The amount of the polymerization initiator to be used is generally 1 mole with respect to 2 to 100 moles of the cyclic imino ether (12).

As a polymerization solvent, for example, acetic acid esters such as ethyl acetate and propyl acetate, ethers such as diethyl ether, diisopropyl ether, dioxane, and tetrahydrofuran, ketones such as acetone and methyl ethyl ketone, halogen solvents such as chloroform and methylene chloride, nitrile solvents such as acetonitrile and benzonitrile, aprotonic polar solvents such as N,N-dimethyl formamide, N,N-dimethylacetamide, and dimethyl sulfoxide may be used. Among them, acetic esters can be suitably used. The amount of the solvent to be used is generally 20 to 2000 parts by mass with respect to 100 parts by mass of the cyclic imino ether (4).

The polymerization temperature is generally from 30 to 170°C, and preferably from 40 to 150°C, and the polymerization time is depending upon the polymerization temperature etc. and generally from 1 to 60 hour(s).

In the case that, for example, 2-substituted-2-oxazoline is used as the cyclic imino ether (12), poly(N-acylalkyleneimine) wherein n is 2 in the above-mentioned formula (9) is obtained. In the case that 2-substituted-dihydro-2-oxazin is used, poly(N-acylpropyleneimine) wherein n is 3 in the above-mentioned formula (9) is obtained.

Method for coupling between poly(N-acylalkyleneimine) and organopolysiloxane segment includes, for example,

1) Reaction between the modified organopolysiloxane represented by the above-mentioned formula (11) and terminal reactive poly(N-acylalkyleneimine) obtained by living polymerization of cyclic imino ether.
2) Esterification reaction by condensation between a carboxyl group and a hydroxyl group.
3) Amidation reaction by condensation between a carboxyl group and an amino group.
4) Formation reaction of secondary, tertiary or quarternary ammonium by reaction between a halogenated alkyl group and a primary, secondary or tertiary amino group.

5) Addition reaction of a vinyl group to organopolysiloxane having a Si-H group.
6) β-hydroxyamine formation reaction between an epoxy group and an amino group.

Among them, the above-mentioned method 1) is more* effective, because control of the degree of polymerization is easy by adjusting the amount of use of the cyclic imino ether (12) and the polymerization initiator according to the below theoretical formula (wherein MWi denotes the molecular weight of poly(N-acylalkyleneimine)), and moreover, poly (N-acylalkyleneimine) having a narrow, nearly monodispersed molecular weight distribution than that of the usual radical polymerization can be obtained.
$$MWi = \frac{\text{Number of moles of cyclic imino ether}}{\text{Number of moles of polymerization initiator}} \times \text{Molecular weight of cyclic imino ether} + \text{Molecular weight of polymerization initiator}$$

Examples of the organopolysiloxane as the component (D) include poly(N-formylethyleneimine)organosiloxane, poly(N-acetylethyleneimine)organosiloxane, and poly(N-propionylethyleneimine)organosiloxane.
The organopolysiloxane of the component (D) can be used alone or in combination of two or more, and the content in the whole composition is preferably from 0.1 to 10 mass%, more preferably from 2.1 to 8 mass%, and even more preferably from 4.2 to 6 mass% from the viewpoints of the hair setting performance, hair-re-styling performance, and washing property when washing hair.
The ratio (C)/[(A)+(B)+(D)], the ratio of the content of the component (C) to the total content of the components (A), (B) and (D), is preferably adjusted in the range from 0.1 to 3, more preferably in the range from 0.2 to 2, and even more preferably in the range from 0.3 to 1.5 from the viewpoint that the hairspray of the present invention is provided evenly with keeping ability and being free from stiffness and stickiness.

[(E): Liquid, Nonionic Surfactants having HLB value of 2.8 to 20]

The hairspray of the present invention may further contain as the component (E) nonionic surfactants which have HLB values of 2.8 to 20 and which are in a liquid state at 30°C. The component (E) improves the miscibility of the components (A) to (D) and functions as a plasticizer of the component (A). Examples of such nonionic surfactants include oleic acid monoglyceride (HLB2.8), palm kernel fatty acid diethanolamide (HLB2.9), caprylic acid monoglyceride (HLB3.2), sorbitan monooleate (HLB4.3), isostearyl glyceryl ether (HLB5.0), sorbitan monolaurate (HLB8.6), polyoxyethylene sorbitol tetraoleate (HLB10.5), polyoxyethylene sorbitan trioleate (HLB11.0), polyoxyethylene sorbitol tetraoleate (HLB11.8), polyoxyethylene sorbitan monooleate (HLB13.3), polyoxyethylene (9) tridecyl ether (HLB13.3), polyoxyethylene sorbitan monostearate (HLB14.9), and polyoxyethylene sorbitan monopalmitate (HLB15.6). Among them, isostearyl glyceryl ether, and polyoxyethylene sorbitan monooleate are preferred. The HLB used herein means a calculated value by Griffin's method.
These nonionic surfactants can be used alone or in combination of two or more as the component (E), and the content in the whole composition is preferably from 0.1 to 20 mass%, more preferably from 0.2 to 10 mass%, and even more preferably from 0.3 to 5 mass%.
The ratio (E)/[(A)+(B)+(C)+(D)], the ratio of the content of the component (E) to the total content of the components (A) to (D), is preferably adjusted in the range from 0.01 to 0.5, more preferably in the range from 0.03 to 0.3, and even more preferably in the range from 0.05 to 0.1 from the viewpoint that the hairspray of the present invention is provided with hair-re-styling ability and no stickiness.

[Solvent]

It is preferable that the hairspray of the present invention further contains a lower alcohol having 4 or less carbon atoms (for example, ethanol) and/or water as a solvent. The content of the solvent with respect to the whole composition is preferably from 4 to 98.4 mass%, more preferably from 30 to 98 mass%, and even more preferably from 50 to 95 mass% from the viewpoint of improving the solubility and spray property of the copolymer and a plasticizer contained in a cosmetic composition.

[Other Components]

The hairspray of the present invention may contain various components used in ordinary hair cosmetic compositions such as cationic surfactants, anionic surfactants, nonionic surfactants other than those described above,

amphoteric surfactants, pH regulators, vitamin preparations, proteins, amino acids, crude drugs, antiseptics, ultraviolet absorbers, antioxidants, and colorants, depending on the intended use.

[Dosage Forms and others]

The hairspray of the present invention may be either an aerosol spray type or a pump spray type.

When the hairspray of the present invention is an aerosol hairspray, a propellant is further used. The aerosol hairspray is manufactured by filling components other than the propellant (aerosol stock solution) and a propellant in a pressure container.

Examples of the propellant include, for example, liquefied petroleum gas (LPG), dimethyl ether (DME), carbon dioxide gas, a nitrogen gas, and the mixture thereof. Furthermore, chlorofluorocarbon alternative such as HFC-152a can be used. The amount of the propellant (expressed by the mass ratio of the stock solution to the propellant, stock solution/propellant), is preferably in the range from 5/95 to 80/20, more preferably in the range from 30/70 to 70/30, and even more preferably in the range from 40/60 to 60/40 in order to obtain preferable usability, hairdressing performance, and hair-re-styling performance and to obtain preferable spray property and adhesive property. Furthermore, in order to obtain preferable spray property and preferable adhesive property, a pressure in the pressure container is preferably adjusted to from 0.12 to 0.45 MPa at 20°C.

The viscosity of the stock solution is preferably, at 30°C, 15 mPa·s or less, more preferably 10 mPa·s or less in order to spray the stock solution as fine droplets. The term "viscosity" as used herein means a value measured by using a Brookfield viscometer (Rotor with BL adapter, rotation speed at 30 rpm, for 60 seconds at 30°C).

A valve to be used for the pressure container has preferably a stem diameter $\phi$ of from 0.33 to 0.46 mm and a housing diameter $\phi$ of from 0.33 to 0.65 mm x a vapor tap diameter $\phi$ of from 0 to 0.64 mm. In the case of a water-containing formulation, the valve has more preferably a stem diameter $\phi$ of from 0.33 to 0.42 mm and a housing diameter $\phi$ of from 0.33 to 0.42 mm and has no vapor tap, while in the case of a non-aqueous formulation, the valve has more preferably a stem diameter $\phi$ of from 0.40 to 0.46 mm and a housing diameter $\phi$ of from 0.42 to 0.65 mm x a vapor tap diameter $\phi$ of from 0.33 to 0.46 mm.

## Examples

### Synthesis Example 1: Organopolysiloxane A

0.8 g (0.005 mol) of Diethyl sulfate and 12.8 g (0.14 mol) of 2-ethyl-2-oxazoline were dissolved in 29 g of dehydrated ethyl acetate and the resulting solution was heated under reflux for 8 hours in a nitrogen atmosphere to yield a terminal-reactive poly(N-propionylethyleneimine). The number average molecular weight, measured by GPC, was 2700. A 33% solution of 100 g of side-chain primary aminopropyl-modified polydimethylsiloxane (weight average molecular weight: 100000, amine equivalent: 20000) in ethyl acetate was added at one time and the resulting mixture was heated under reflux for 10 hours. The reaction mixture was concentrated under reduced pressure to yield an N-propionylethyleneimine-dimethylsiloxane copolymer as a pale yellow rubber-like solid (111 g, yield: 98%). The content of organopolysiloxane segment in the final product was 88 mass% and weight average molecular weight was 114000. As a result of neutralization titration with hydrochloric acid (methanol was used as a solvent), no amino group remained.

### Synthesis Example 2: Organopolysiloxane B

Similar to the above Synthesis Example 1, poly(N-propionylethyleneimine) having a number average molecular weight of 1100 was obtained using 19.0 g (0.12 mol) of diethyl sulfate, 81.0 g (0.82 mol) of 2-ethyl-2-oxazoline, and 203.0 g of dehydrated ethyl acetate. Furthermore, an N-propionylethyleneimine-dimethylsiloxane copolymer was obtained as a pale yellow rubber-like solid (390 g, yield 97%) by using 300 g of side-chain primary aminopropyl-modified polydimethylsiloxane (weight average molecular weight: 32000, amine equivalent: 2000). The content of silicone segment in the final product was 75 mass% and weight average molecular weight was 40000. As a result of neutralization titration with hydrochloric acid (methanol was used as a solvent), about 20 mol% of amino groups remained.

### Synthesis Example 3: Organopolysiloxane C

Similar to the above Synthesis Example 1, poly(N-propionylethyleneimine) having a number average molecular weight of 1300 was obtained using 6.5 g (0.042 mol) of diethyl sulfate, 34.4 g (0.36 mol) of 2-ethyl-2-oxazoline, and 87 g of dehydrated ethyl acetate. Furthermore, an N-propionylethyleneimine-dimethylsiloxane copolymer was obtained as a pale yellow rubber-like semi-solid (138 g, yield: 98%) by using 100 g of side-chain primary aminopropyl-modified polydimethylsiloxane (weight average molecular weight: 32000, amine equivalent: 2000). The content of organopolysiloxane segment in the final product was 71 mass% and a weight average molecular weight was 46000. As a result of neutralization titration with hydrochloric acid (methanol was used as a solvent), about 22 mol% of amino groups remained.

Synthesis Example 4: Organopolysiloxane D

Similar to the above Synthesis Example 1, poly(N-propionylethyleneimine) having a number average molecular weight of 5200 was obtained using 3.2 g (0.021 mol) of diethyl sulfate, 92.8 g (0.98 mol) of 2-ethyl-2-oxazoline, and 205 g of dehydrated ethyl acetate. Furthermore, an N-propionylethyleneimine-dimethylsiloxane copolymer was obtained as a pale yellow rubber-like solid (188 g, yield: 96%) by using 100 g of side-chain primary aminopropyl-modified polydimethylsiloxane (weight average molecular weight: 50000, amine equivalent: 3800). The content of organopolysiloxane segment in the final product was 51 mass% and a weight average molecular weight was 98000. As a result of neutralization titration with hydrochloric acid (methanol was used as a solvent), about 24 mol% of amino groups remained.

Examples 1 to 13 and Comparative Examples 1 to 4

According to the standard method, hairspray stock solutions shown in Table 1 were prepared. Each of the stock solutions and LPG (0.27 MPa, 20°C) as a propellant were filled in an aerosol container equipped with the below-described valve and button at a stock solution/propellant ratio (mass ratio) of 55/45.
Valve: stem diameter ϕ: 0.40 mm, housing diameter ϕ: 0.65 mm ×vapor tap diameter ϕ: 0.42 mm
Button: orifice diameter ϕ: 0.43 mm (Mitani Valve)
The hairspray was evaluated for "keeping ability," "free from stiffness," "free from stickiness," and "hair-re-styling performance." The results are shown in Table 1.
"Keeping ability," "free from stiffness," " "free from stickiness," "hair-re-styling performance," and "overall evaluation"

(Evaluation Method)

Ten expert panel members set hair by using each hairspray, and carried out sensory evaluation for "keeping ability," "free from stiffness," and "free from stickiness."
Furthermore, nine hours after hair dressing in the environment at 25°C and 65%, they also carried out the sensory evaluation for "hair-re-styling performance. Note here that the evaluation was carried out according to the below-mentioned criteria, and the evaluation was expressed by a mean value of evaluation scores of the ten panel members.
Furthermore, the mean value of the above evaluation was expressed as "overall evaluation."

(Evaluation Criteria)

5: Excellent.

4: Good.

3: Fairly good.

2: Fairly poor.

1: Poor.

[Table 1]

| (mass%: all numeric values show active values) | | Examples | | | | | | | | | | | | | Comparative Examples | | | |
|---|---|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 1 | 2 | 3 | 4 |
| (A) | (Acrylamide/DMAPA acrylate/methoxy PEG methacrylate) copolymer (*1) | 1.52 | 1.52 | 0.96 | | | 1.52 | 0.87 | 0.87 | | 0.65 | | 0.65 | | 6.00 | | 0.04 | 0.96 |
| | (Acrylamide/alkyl acrylate/DMAPA acrylate/methoxy PEG methacrylate) copolymer (*2) | | | | 0.96 | 0.96 | | | | 0.76 | | 2.18 | | 1.52 | | 4.11 | | |
| (B) | Acrylates/$C_{1-18}$ alkyl acrylates/$C_{1-8}$ alkylacrylamide copolymer neutralized with 2-amino-2-methyl-1-propyl alcohol (*3) | 5.06 | | 3.21 | 3.21 | | 5.06 | 2.89 | 2.89 | 2.53 | 3.52 | 1.99 | 2.17 | 5.06 | | 0.31 | 4.38 | 3.21 |
| | Acrylates/diacetoneacrylamide copolymer neutralized with 2-amino-2-methyl-1-propyl alcohol (*4) | | 5.06 | | | 3.21 | | | | | | | | | | | | |
| (C) | dipropylene glycol | 1.90 | 1.90 | 3.34 | 3.34 | 3.34 | 2.38 | 4.56 | 5.70 | 4.94 | 3.34 | 3.34 | 5.32 | 0.95 | 4.00 | 3.34 | 3.34 | |
| | 1,3-butylene glycol | 0.48 | 0.48 | 0.95 | 0.95 | 0.95 | | 1.14 | | 1.24 | 0.95 | 0.95 | 1.33 | 0.95 | | 0.95 | 0.95 | |
| (D) | Organopolysiloxane A of Synthesis Example 1 | | | | | | | 0.04 | | | | | | | | | | |
| | Organopolysiloxane B of Synthesis Example 2 | | | 0.10 | | | | | | | | | | | | | | |
| | Organopolysiloxane C of Synthesis Example 3 | | | | | | | | | | | 0.10 | | | | | | |
| (E) | isostearyl glyceryl ether | 0.48 | 0.48 | 0.79 | 0.79 | 0.79 | 0.48 | | | | 0.79 | | | 0.95 | 1.00 | 0.79 | 0.79 | 5.08 |
| | polyoxyethylene (9) tridecyl ether | | | | | | | | | | | 0.79 | | | | | | |
| Others | neopentyl glycol dicaprate | | | 0.15 | 0.15 | 0.15 | | | | 0.10 | | | 0.20 | | | | | 0.15 |
| | lactic acid | 0.02 | 0.02 | 0.01 | 0.08 | 0.08 | 0.02 | 0.01 | 0.01 | 0.07 | 0.01 | 0.19 | 0.01 | 0.13 | 0.07 | 0.36 | | 0.01 |
| | ethanol | balance | balance | balance | balance | balance | balance | balance | balance | balance | balance | balance | balance | balance | balance | balance | balance | balance |
| Mass ratio | (B)/(A) | 3.33 | 3.33 | 3.34 | 3.34 | 3.34 | 3.33 | 3.32 | 3.32 | 3.33 | 5.42 | 0.91 | 3.34 | 3.33 | 0.00 | 0.08 | 109.5 | 3.34 |
| | (C)/[(A)+(B)+(D)] | 0.36 | 0.36 | 1.00 | 1.03 | 1.03 | 0.36 | 1.50 | 1.52 | 1.88 | 1.03 | 1.00 | 2.36 | 0.29 | 0.67 | 0.97 | 0.97 | 0.00 |
| | (E)/[(A)+(B)+(C)+(D)] | 0.05 | 0.05 | 0.09 | 0.09 | 0.09 | 0.05 | 0.00 | 0.00 | 0.00 | 0.09 | 0.09 | 0.00 | 0.11 | 0.10 | 0.09 | 0.09 | 1.22 |
| Evaluation | keeping ability | 4.7 | 4.6 | 4.4 | 4.5 | 4.6 | 4.8 | 3.6 | 3.5 | 3.6 | 4.3 | 4.2 | 3.3 | 4.6 | 2.4 | 3.7 | 4.1 | 3.8 |
| | free from stiffness | 3.6 | 3.6 | 4.3 | 4.0 | 3.7 | 3.3 | 4.7 | 4.8 | 4.8 | 2.7 | 3.4 | 4.6 | 2.6 | 4.2 | 2.4 | 1.6 | 2.6 |
| | free from stickiness | 4.7 | 4.6 | 4.4 | 4.2 | 4.0 | 4.5 | 4.2 | 4.0 | 3.7 | 4.5 | 3.4 | 2.8 | 3.6 | 3.5 | 3.4 | 4.2 | 1.7 |
| | hair-re-styling performance | 3.5 | 3.9 | 4.5 | 4.1 | 4.5 | 3.9 | 3.7 | 3.7 | 3.7 | 4.4 | 4.3 | 3.6 | 3.7 | 3.6 | 3.7 | 3.5 | 4.4 |
| | overall evaluation | 4.1 | 4.2 | 4.4 | 4.2 | 4.2 | 4.1 | 4.1 | 4.0 | 4.0 | 4.0 | 3.8 | 3.6 | 3.6 | 3.4 | 3.3 | 3.4 | 3.1 |

*1: N-tert-butylacylamide/dimethylacrylamide/dimethylamainopropylacry lamide/methoxypolyethylene glycol (PEG400) methacrylate copolymer <52/25/2/21> (mass ratio) synthesized in accordance with the process described in JP-A-08-291206, weight average molecular weight: about 120000

*2: N-tert-butylacrylamide/ethyl acrylate/N,N-dimethylaminopropylacrylamide/polyethylene glycol methacrylate copolymer <55/20/15/10> (mass ratio) described in JP-A-02-180911, weight average molecular weight: about 120000

*3: Plascize L-9909B (manufactured by Goo Chemical)

*4: Plascize L-9540B (manufactured by Goo Chemical)

Formulation Example 1

The hairspray stock solution with the below-mentioned formulation was prepared according to the standard method, and was filled together with LPG (0.27 MPa, 20°C) as a propellant in the aerosol container similar to the above Example 1 at the ratio of stock solution/propellant (weight ratio) of 55/45.

| Stock solution composition | (weight%) |
|---|---|
| (Acrylamide/DMAPA acrylate/methoxy PEG methacrylate) copolymer (*1) | 0.96 |
| Acrylates/$C_{1-18}$ alkyl acrylates/$C_{1-8}$ alkyl acrylamide copolymer neutralized with 2-amino-2-methyl-1-propyl alcohol (*3) | 3.21 |
| Dipropylene glycol | 3.34 |
| 1,3-Butylene glycol | 0.95 |
| Organopolysiloxane D of Synthesis Example 4 | 0.10 |
| Isostearyl glyceryl ether | 0.79 |
| Neopentyl glycol dicaprate | 0.15 |
| Lactic acid | 0.01 |
| Ethanol | balance |

The obtained hairspray was excellent in the keeping ability, free from stiffness, free from stickiness, and hair-re-styling performance .

Formulation Example 2

A hair cosmetic composition having the below-mentioned prescription was filled in a container according to the standard method to produce a pump spray (Y-150 sprayer manufactured by Yoshino Kogyosho Co., Ltd. was used).

| Stock solution composition | (weight%) |
|---|---|
| (Acrylamide/DMAPA acrylate/methoxy PEG methacrylate) copolymer (*1) | 0.96 |
| Acrylates/$C_{1-18}$ alkyl acrylates/$C_{1-8}$ alkyl acrylamide copolymer neutralized with 2-amino-2-methyl-1-propyl alcohol (*3) | 3.21 |
| Dipropylene glycol | 3.34 |
| 1,3-butylene glycol | 0.95 |
| Organopolysiloxane B of Synthesis Example 2 | 0.10 |
| Isostearyl glyceryl ether | 0.79 |
| Neopentyl glycol dicaprate | 0.15 |
| Lactic acid | 0.01 |
| Ethanol | balance |

The obtained pump hairspray was excellent in the keeping ability, free from stiffness, free from stickiness, and hair-re-styling performance.

## Claims

**1.** A hairspray comprising the following components (A) to (C):

(A) one or more cationic coating film-forming polymers selected from (A1) and (A2);

(A1) a copolymer obtained from a monomer mixture containing the following monomers (a) to (d):

(a) 30 to 80 mass% of a (meth)acrylamide monomer represented by a formula (1):

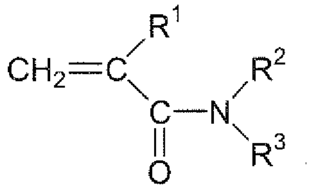

(1)

wherein $R^1$ is a hydrogen atom or a methyl group, $R^2$ and $R^3$ are the same as or different from each other and are a hydrogen atom or an alkyl group having 4 to 12 carbon atoms, with the proviso that both $R^2$ and $R^2$ are not a hydrogen atom simultaneously,

(b) 2 to 50 mass% of a (meth)acrylamide monomer represented by a formula (2):

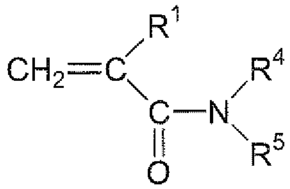

(2)

wherein $R^1$ is the same as mentioned above, $R^4$ and $R^5$ are the same as or different from each other and are a hydrogen atom or an alkyl group having 1 to 3 carbon atoms,

(c) 0 to 30 mass% of a (meth)acrylic ester monomer or a (meth)acrylamide monomer represented by

a formula (3):

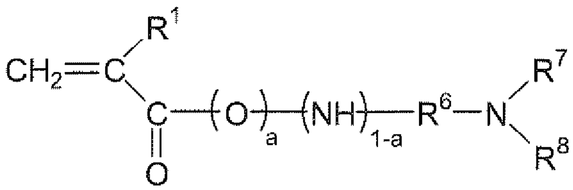

(3)

wherein $R^1$ is the same as mentioned above, $R^6$ is an alkylene group having 2 or 3 carbon atoms, $R^7$ and $R^8$ are the same as or different from each other and are a methyl group or an ethyl group, and "a" is a number of 0 or 1, and

(d) 0 to 40 mass% of a (meth)acrylic ester monomer represented by a formula (4):

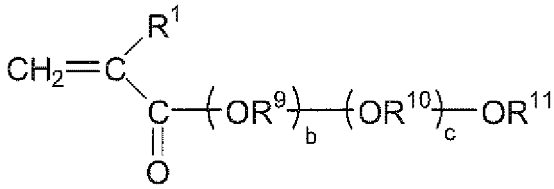

(4)

wherein $R^1$ is the same as mentioned above, $R^9$ and $R^{10}$ are the same as or different from each other and are an alkylene group having 2 to 4 carbon atoms, $R^{11}$ is a hydrogen atom, an alkyl group having 1 to 10 carbon atoms or a phenyl group, and "b" and "c" are independently a number from 0 to 50, with the proviso that both "b" and "c" are not 0 simultaneously; and

(A2) a copolymer obtained from a monomer mixture containing the following monomers (e) to (h):

(e) 30 to 80 mass% of a (meth)acrylamide monomer represented by a formula (5):

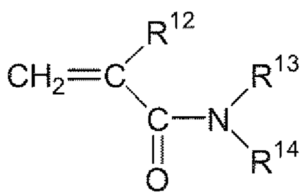

(5)

wherein $R^{12}$ is a hydrogen atom or a methyl group, $R^{13}$ and $R^{14}$ are the same as or different from each other and are a hydrogen atom or an alkyl group having 4 to 12 carbon atoms, or $R^{13}$ and $R^{14}$ are combined, together with an adjacent nitrogen atom, to form a ring,

(f) 5 to 45 mass% of a (meth)acrylic ester monomer represented by a formula (6):

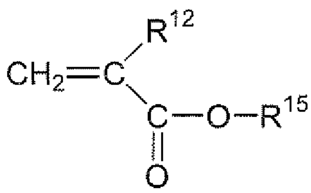

(6)

wherein $R^{12}$ is the same as mentioned above, and $R^{15}$ is an alkyl group having 1 to 4 carbon atoms,

(g) 2 to 30 mass% of a (meth)acrylic ester monomer or a (meth)acrylamide monomer represented by a formula (7):

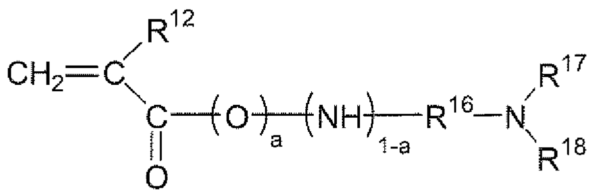

(7)

wherein $R^{12}$ is the same as mentioned above, $R^{16}$ is an alkylene group having 2 or 3 carbon atoms, $R^{17}$ and $R^{18}$ are the same as or different from each other and are a methyl group or an ethyl group, and "a" is a number of 0 or 1, and

(h) 0 to 30 mass% of a (meth)acrylic ester monomer represented by a formula (8):

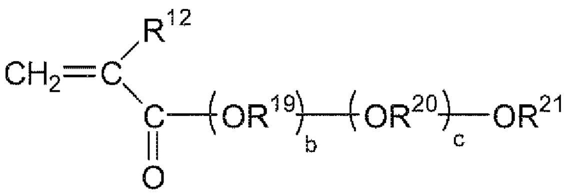

(8)

wherein $R^{12}$ is the same as mentioned above, $R^{19}$ and $R^{20}$ are the same as or different from each other and are an alkylene group having 2 to 4 carbon atoms, $R^{21}$ is a hydrogen atom or a methyl group, and "b" and "c" are independently a number of from 0 to 50, with the proviso that both "b" and "c" are not 0 simultaneously;

(B) an anionic coating film-forming polymer; and

(C) a solvent which has two or more hydroxyl groups, has a molecular weight of 62 or more and 1000 or less, and is in a liquid state at 30°C,

and wherein a mass ratio (B)/(A) of the component (B) to the component (A) is from 0.1 to 100.

**2.** The hairspray according to claim 1, further comprising poly(N-acylalkyleneimine)-modified silicone as a component (D), wherein a mass ratio of the components (A) to (D), (C)/[(A)+(B)+(D)], is in a range from 0.1 to 3.

**3.** The hairspray according to claim 1 or 2, further comprising a nonionic surfactant which has HLB of 2.8 to 20 and is in a liquid state at 30°C as a component (E).

**4.** The hairspray according to any one of claims 1 to 3, wherein a content of the component (A) is 0.3 to 10 mass% with respect to a whole composition of the hairspray.

**5.** The hairspray according to any one of claims 1 to 4, wherein a content of the component (B) is 0.5 to 12 mass% with respect to the whole composition of the hairspray.

**6.** The hairspray according to any one of claims 1 to 5, wherein a content of the component (C) is 1 to 25 mass% with respect to the whole composition of the hairspray.

**7.** The hairspray according to any one of claims 2 to 6, wherein a content of the component (D) is 0.1 to 10 mass% with respect to the whole composition of the hairspray.

**8.** The hairspray according to any one of claims 3 to 7, wherein a content of the component (E) is 0.1 to 20 mass% with respect to the whole composition of the hairspray.

**9.** The hairspray according to any one of claims 2 to 8, wherein
the poly(N-acylalkyleneimine)-modified silicone of the component (D) is an organopolysiloxane wherein the poly(N-acylalkyleneimine) segment comprising repeating units each represented by the following formula (9) is linked to at least two of silicon atoms of an organopolysiloxane segment constituting a main chain via a heteroatom-containing alkylene group:

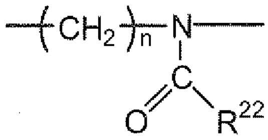

(9)

wherein, $R^{22}$ is a hydrogen atom, an alkyl group having 1 to 22 carbon atoms, an aralkyl group, or an aryl group, and n is 2 or 3,
a number average molecular weight of the poly(N-acylalkyleneimine) segment is 500 to 10,000,
a mass ratio (a/b) of the organopolysiloxane segment (a) constituting a main chain and the poly(N-acylalkyleneimine) segment (b) is 40/60 to 98/2, and
a weight average molecular weight of the organopolysiloxane segment constituting a main chain is 10,000 to 150,000.

**10.** The hairspray according to any one of claims 1 to 9, wherein
the anionic coating film-forming polymer of the component (B) is selected from the group consisting of
an anionic coating film-forming polymer containing any of crotonic acid, maleic acid, maleic acid monoester, and itaconic acid, as a structural unit;
an anionic coating film-forming polymer containing (meth)acrylic acid as a structural unit;
a natural anionic coating film-forming polymer having either of a carboxyl group and a sulfuric acid group; and
a polyester including either of a carboxyl group and a sulfonic acid group.

## INTERNATIONAL SEARCH REPORT

International application No.
PCT/JP2010/003481

A. CLASSIFICATION OF SUBJECT MATTER
*A61K8/81*(2006.01)i, *A61K8/34*(2006.01)i, *A61K8/898*(2006.01)i, *A61Q5/06*(2006.01)i, *C08F20/56*(2006.01)i, *C08F220/10*(2006.01)i, *C08F220/56*(2006.01)i

According to International Patent Classification (IPC) or to both national classification and IPC

B. FIELDS SEARCHED

Minimum documentation searched (classification system followed by classification symbols)
A61K8/81, A61K8/34, A61K8/898, A61Q5/06, C08F20/56, C08F220/10, C08F220/56

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 8-291206 A (Kao Corp.), 05 November 1996 (05.11.1996), claims; paragraphs [0037], [0038] & US 5663261 A & EP 728778 A1 | 1-10 |
| Y | JP 2-180911 A (Kao Corp.), 13 July 1990 (13.07.1990), claims; page 6, upper left column & US 5278269 A & EP 372546 B1 | 1-10 |
| Y | JP 62-7164 B2 (L'Oreal), 16 February 1987 (16.02.1987), claims; columns 23 to 24, 97 & US 4240450 A & GB 1603321 A | 1-10 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 11 June, 2010 (11.06.10) | 22 June, 2010 (22.06.10) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2010/003481

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2008/064971 A1 (UNILEVER N.V.), 05 June 2008 (05.06.2008), claims; pages 1, 6, 11 & US 2010/61954 A1 & EP 2099427 A1 | 1-10 |
| Y | JP 2005-68134 A (Kao Corp.), 17 March 2005 (17.03.2005), claims; paragraphs [0005], [0012], [0013], [0016] & US 2005/63916 A1 & EP 1504744 A1 | 1-10 |
| Y | JP 2009-24114 A (Kao Corp.), 05 February 2009 (05.02.2009), claims; paragraphs [0005], [0042] to [0045], [0056] & EP 2170258 A1 | 2-10 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005068134 A
- JP 2009007347 A
- JP 8291206 A
- JP 2180911 A